# FASCICULE DE BREVET EUROPEEN

(11) **EP 0 739 985 B1**
(45) Date de publication et mention de la délivrance du brevet: **15.11.2000**
(21) Numéro de dépôt: 96200827.2
(22) Date de dépôt: 27.03.1996
(51) Int. Cl.: C12P 9/00, C12N 11/18, A23D 7/01, A23J 7/00, A23L 1/24

(54) **Procédé d'interestérification de phospholipides**
Verfahren zur Interesterifikation von Phospholipiden
Process for phospholipid interesterification

(30) Priorité: 24.04.1995 US 427544
(43) Date de publication de la demande: 30.10.1996
(73) Titulaire: SOCIETE DES PRODUITS NESTLE S.A., 1800 Vevey (CH)
(72) Inventeur: Chmiel, Oliver, 1350 Orbe (CH); Melachouris, Nicholas, 1125 Monnaz (CH); Traitler, Helmut, 1802 Corseaux (CH)
(74) Mandataire: Archambault, Jean

(56) Documents cités:
- EP-A- 0 378 893
- WO-A-91/03564
- BIOCATALYSIS, vol. 9, 1994, pages 181-194, XP000577906 A. MUSTRANTA ET AL.: "Modification of phospholipids with lipases and phospholipases"
- DATABASE WPI Section Ch, Week 8306 Derwent Publications Ltd., London, GB; Class D13, AN 83-12989k XP002011040 "Instant coffee preparation by coating fine coffee powder with edible polyhydric alcohol(fatty acid ester) or phospholipid and mixing with spray dried aqueous coffee extract" & JP-A-57 208 947 (CALPIS SHOKUHIN KOG) , 22 Décembre 1982

## Description

La présente invention a pour objet un procédé pour échanger des groupes acyle dans un phospholipide par échange enzymatique d'ester avec un triacyl-glycérol.

Les glycérophospholipides tels que la phosphatidylcholine consistent en glycérol estérifié avec deux groupes acyle d'acide gras et un groupe phosphate ou phosphate estérifié. Pour certaines applications des phospholipides, il est souhaitable d'échanger les groupes acyle présents dans les phospholipides, par exemple afin d'améliorer leur stabilité thermique.

A cet égard, il a déjà été montré, par exemple dans le document US-A-5 314 706, que le jaune d'oeuf additionné de lysophosphatidylcholine exogène obtenue par hydrolyse de la lécithine avec une phospholipase A2 a amélioré la thermostabilité d'émulsions, en particulier de la mayonnaise.

En outre, il a été montré dans le document WO 91/03564 que l'interestérification enzymatique d'un phospholipide avec un acide gras au moyen d'un catalyseur consistant en une lipase immobilisée sur un support macroporeux dans un solvant organique a provoqué une incorporation améliorée de groupement acyle spécifiques dans le phospholipide. Dans ce procédé, il a été utilisé un large excès d'acides gras qui persiste dans le mélange réactionnel à la fin de la mise en oeuvre du procédé et qui n'est pas aisément séparé de la lécithine modifiée désirée.

Dans "Biocatalysis" 1994, Vol. 9, pages 181-194, on décrit entre autres un procédé de transestérification de phospholipide consistant à échanger certains acides gras du phospholipide contre des acides gras libres dans une lipolyse enzymatique en présence d'une lipase ou d'une phospholipase immobilisée. A la page 190, dernier paragraphe, il est question de transestérification d'un phospholipide synthétique contenant deux restes d'acide myristique en présence d'un large excès d'acide oléique par rapport au substrat phospholipide au moyen d'une lipase (lipozyme ou lipase d'A.niger) en l'absence de solvant et en présence de 5 % d'eau (lignes 2-8). Il est indiqué plus loin que la phospholipase est inactive dans la réaction de transestérification sans solvant (lignes 11-12). Dans le passage reliant les pages 190 et 191, il est spécifié qu'un degré important d'hydrolyse se produit à côté de la transestérification. Si la transestérification échange les acides gras, il est clair que l'hydrolyse libère une quantité supplémentaire d'acide gras, qui vient s'ajouter à l'excès mis en oeuvre. En conclusion, les auteurs suggèrent que la transestérification doit s'effectuer préférablement au moyen de lipase en présence d'eau ainsi que d'un large excès d'acide gras.

Un objectif de la présente invention consiste à modifier la composition en acides gras des phospholipides pour remplacer les acides gras insaturés à chaîne longue par des acides gras à chaîne courte et à chaîne moyenne.

De manière tout à fait inattendue, il s'est révélé à présent possible d'effectuer l'interestérification de triglycérides avec des phospholipides en présence d'une lipase immobilisée et d'une phospholipase immobilisée, de manière à incorporer des groupements acides gras à chaîne courte aux positions 1 et 2 du groupement glycérol. Aucune augmentation importante de la quantité d'acides gras libres n'a été détectée à un quelconque moment au cours de la réaction. En outre, il était inattendu que la lécithine modifiée soit un meilleur émulsionnant que la lécithine naturelle pour certaines formulations d'émulsions telles que, par exemple, la mayonnaise et l'assaisonnement pour salades. De plus, la mouillabilité des poudres, en particulier les poudres de lait et de cacao additionnées de lécithine, a été considérablement améliorée lorsque de la lécithine modifiée conformément au procédé de la présente invention a été utilisée. Un autre avantage décisif des lécithines modifiées obtenues par le procédé de la présente invention est leur absence d'une quelconque saveur désagréable.

Le procédé conforme à la présente invention est caractérisé en ce que la réaction s'effectue en l'absence de solvant, avec un système enzymatique consistant en un mélange d'une lipase immobilisée et d'une phospholipase immobilisée et que le triacylglycérol contient des groupes acyle saturés à chaîne courte en C₂ à C₄ et/ou moyenne en C6 à C12.

Le procédé de la présente invention peut être appliqué à n'importe quel type désiré de phospholipides contenant des groupes esters à fonction acyle d'acides gras. Des exemples de ces phospholipides naturels sont l'acide phosphatidique, la phosphatidylcholine, la phosphatidylsérine, le phosphatidylglycérol, le phosphatidylinositol, la phosphatidyléthanolamine et le diphosphatidylglycérol. Des phospholipides synthétiques avec divers composés hydroxyliques estérifiés en le groupe phosphate, les 1-alkyl-2-acylphospholipides et des diacylphospholipides peuvent également être traités.

La réaction d'échange peut être utilisée pour incorporer le groupement acide gras désiré à un phospholipide. Les groupements visés sont des acides gras saturés à chaîne courte en C2 à C4, et les groupements acides gras saturés à chaîne moyenne en C6 à C12. Toutes les huiles et matières grasses contenant une quantité importante de ces groupements acides gras incorporée aux triglycérides, notamment des triglycérides à chaîne courte, à chaîne moyenne et l'huile de beurre, de préférence la triacétine, peuvent être utilisées comme matières de départ.

Le catalyseur enzymatique utilisé comprend une lipase qui peut être d'origine animale, végétale ou microbienne et qui peut être non spécifique ou spécifique du point de vue de la position, par exemple Lypozyme (TM), Novo Nordisk a/s. Il comprend également une phospholipase, de préférence une phospholipase extracellulaire A2, par exemple Lecitase (TM), Novo Nordisk a/s.

Les enzymes utilisés dans le procédé de la présente invention sont immobilisés sur un support organique ou inorganique macroporeux en particules, et sont de préférence fixés au support par réticulation avec n'importe quel agent de réticulation convenable, par exemple le glutaraldéhyde.

Le rapport de la lipase immobilisée à la phospholipase immobilisée est choisi de telle sorte que la lipase représente 25 à 75 % et de préférence 30 à 70 % en poids de la quantité totale de billes d'enzyme, ce qui correspond également au même pourcentage d'activité totale du système enzymatique.

Le procédé d'interestérification doit être mis en oeuvre dans des conditions dans lesquelles une activité et une thermostabilité optimales des enzymes immobilisés sont obtenues, avantageusement à une température de 60 à 80°C pendant un temps de 1 à 72 heures, de préférence pendant environ 23 heures. A la fin de la réaction, les enzymes sont séparés, par exemple par filtration. Un avantage du procédé de la présente invention consiste en la possibilité aisée, lorsque cela est nécessaire, de séparer les phospholipides des triglycérides, en particulier dans le cas où la triacétine est utilisée comme huile, puisqu'il se produit une séparation de phase immédiate entre la triacétine n'ayant pas réagi et la lécithine.

Dans le cas de n'importe quel autre triglycéride utilisé et lorsqu'une séparation est désirée, les procédés classiques de purification de lécithine, tels que le fractionnement à l'acétone ou le dégommage, par exemple avec de l'acide phosphorique à environ 0,3 % à une température d'environ 90°C, peuvent être mis en oeuvre. A titre de variante qui n'est pas préférée, la séparation peut s'effectuer par chromatographie sur couche mince à hautes performances (CCMHP).

Les lécithines modifiées obtenues par le procédé de la présente invention peuvent être utilisées dans des émulsions alimentaires, par exemple des sauces, la mayonnaise et des assaisonnements pour salades et sont remarquables du fait de leurs propriétés d'émulsionnement améliorées ainsi que de la stabilité thermique des produits auxquels elles sont incorporées.

Elles peuvent également être utilisées dans la production de poudres instantanéisées, par exemple des poudres de lait, de cacao et de café, auxquelles elles confèrent une meilleure mouillabilité que les lécithines ordinaires.

La présente invention est illustrée plus en détail par les exemples suivants, dans lesquels les parties et les pourcentages sont exprimés en poids, sauf spécification contraire.

Dans les exemples, la lipase immobilisée qui est utilisée consistait en Lipozyme (TM), Novo Nordisk a/s.

La phospholipase immobilisée qui a été utilisée était une phospholipase extracellulaire A2 immobilisée sur des billes de verre et préparée de la manière suivante :
une quantité de 0,5 g de billes de verre (aminopropylées, Sigma G-5019) a été placée dans 5 ml d'un tampon au NaH2PO4 100 micromolaire dégazé (pH 7) contenant 2,5 % de glutaraldéhyde et un vide a été maintenu pendant 1 heure. Les billes ont été lavées successivement avec de l'eau, une solution 0,5 molaire de NaCl et une solution 100 millimolaire de NaH2PO4 (pH 6). 1 ml de Lecitase (TM), Novo Nordisk a/s, et 4 ml de ce dernier tampon dégazé ont été ajoutés aux billes et le mélange a été mis en incubation à 4°C pendant une nuit, avec une légère agitation. Après avoir été lavées de nouveau avec ce dernier tampon, les billes ont été stockées dans un tampon d'entreposage.

### Exemple 1

150 g d'un mélange lécithine déshumidifiée:triglycérides à chaîne moyenne en un rapport égal à 1:2 ont été mélangés et chauffés à 70°C. Puis une quantité de 1,5 g de Lipozyme (TM) immobilisé et 0,3 g de la phospholipase A2 immobilisée ont été ajoutés. L'échantillon a été mis en incubation à cette température pendant 23 heures. Les enzymes ont été récupérés par filtration. A des fins d'analyse, les esters méthyliques d'acides gras (FAMES) ont été produits à partir des phospholipides en ajoutant 400 microlitres de chlorure d'acétyle (Fluka) et en conduisant une incubation pendant 20 minutes à 100°C. Les phospholipides ont été séparés de tous les autres constituants par CCMHP (plaques de silicagel 60 F 254, Merk) dans une étape comportant deux migrations, la première avec un mélange toluène:hexane:acide formique en un rapport égal à 70:30:0,5 et ensuite avec un mélange hexane:éther diéthylique:acide formique en un rapport égal à 60:40:1, et ont été extraits du silicagel dans 2 ml d'un mélange hexane:méthanal en un rapport égal à 1:4.

Les FAMES obtenus avec la lécithine de départ et la lécithine modifiée conformément au procédé de la présente invention ont été analysés par chromatographie en phase gazeuse. Les valeurs mentionnées sur le tableau 1 suivant sont basées sur le poids des acides gras.

**TABLEAU 1**

| Acide gras | Lécithine de départ | Lécithine modifiée |
|---|---|---|
| C 4:0 | - | - |
| C 6:0 | - | - |
| C 8:0 | - | 18,4 |
| C 10:0 | 0,1 | 9,1 |
| C 12:0 | - | - |
| C 14:0 | 1,8 | 1,5 |
| C 16:0 | 17,7 | 21,4 |
| C 18:0 | 5,9 | 4,1 |
| C 18:1 | 11,1 | 4,6 |
| C 18:2 | 57 | 35,7 |
| C 18:3 | 6,6 | 4,5 |

A des fins de comparaison, la même lécithine a été traitée avec de la Lecitase (TM) immobilisée (comparaison 1) ou avec du Lipozyme (TM) immobilisé (comparaison 2). La quantité d'acides gras nouveaux incorporée était égale à 18 moles % pour la comparaison 1 et 27 moles % pour la comparaison 2, tandis qu'elle était égale à 45 moles % pour la lécithine modifiée avec le mélange d'enzymes dans le procédé de la présente invention.

### Exemple 2

Le procédé de l'exemple 1 a été utilisé, sauf que la lécithine a été soumise à une réaction avec de la triacétine. Le rapport lécithine déshumidifiée:triacétine était égal à 1:1. Les échantillons ont été abandonnés pendant une nuit pour parvenir à une séparation de phase pratiquement totale. L'enzyme a été récupéré par filtration. La phase supérieure, qui représentait la lécithine, a été analysée pour déterminer la composition en acides gras, de la manière décrite dans l'exemple 1, sauf que, pour une meilleure quantification de C 2:0, des esters butyliques ont été préparés au lieu des esters méthyliques, en utilisant un mode opératoire bien établi dans la littérature.

Les valeurs mentionnées sur le tableau 2 suivant sont basées sur le poids des acides gras.

**TABLEAU 2**

| Acide gras | Lécithine modifiée |
|---|---|
| C 2:0 | 3,5 |
| C 4:0 | 0,3 |
| C 14:0 | 1,9 |
| C 16:0 | 24,5 |
| C 18:0 | 5,4 |
| C 18:1 | 11,3 |
| C 18:2 | 45,5 |
| C 18:3 | 7,7 |

### Exemple 3

Des assaisonnements pour salades contenant 30 % d'huile de soja, 60 % d'eau, 10 % de vinaigre, 0,3 % de gomme xanthane et 1 % de lécithine modifiée de la manière indiquée dans l'exemple 1 ont été préparés. A des fins de comparaison, les mêmes assaisonnements pour salades ont été préparés avec de la lécithine ordinaire (comparaison 3) ou un mélange lécithine ordinaire:triglycéride à chaîne moyenne en un rapport égal à 2:1 (comparaison 4), respectivement.

Leur stabilité thermique a été testée en ce qui concerne l'homogénéité et la viscosité, de la manière suivante :

20 ml de l'assaisonnement respectif pour salades ont été mis en incubation pendant 2 heures à 80°C (température d'un bain d'eau). Après incubation, l'échantillon renfermant la lécithine ordinaire était non homogène, une certaine stratification étant visible, tandis que l'échantillon avec la lécithine modifiée était homogène, sans aucune stratification.

La viscosité a été détectée au moyen d'un essai à la cuillère (écoulement d'un échantillon de la cuillère) et avec un rhéomètre (vitesse de cisaillement par rapport à la tension de cisaillement). Les deux résultats ont mis en évidence une forte viscosité des échantillons renfermant la lécithine modifiée : à une vitesse de cisaillement de 240/s, la tension de cisaillement étant égale à 160 pour l'échantillon renfermant de la lécithine ordinaire et était égale à 185 pour l'échantillon renfermant de la lécithine modifiée. Avant le choc thermique, les deux échantillons avaient une valeur de 185.

Les triglycérides à chaîne moyenne n'ont eu aucune influence sur l'un quelconque de ces facteurs. Les échantillons renfermant la lécithine modifiée conformément à la présente invention se sont révélés être plus thermostables que les échantillons renfermant la lécithine ordinaire (comparaison 3) et les échantillons renfermant le mélange de lécithine ordinaire et de triglycérides à chaîne moyenne. Après centrifugation, une quantité d'huile nettement inférieure a été observée à la partie supérieure, une quantité de 0,25 g d'huile étant recueillie pour une lécithine ordinaire avec (comparaison 4) ou sans (comparaison 3) triglycéride à chaîne moyenne ajouté, comparativement à une quantité de 0,18 g avec la lécithine modifiée conformément à la présente invention. Une réduction nette des saveurs désagréables a été également détectée dans le cas du dernier échantillon.

### Exemple 4

L'effet stabilisant de la lécithine modifiée conforme à la présente invention sur les protéines du jaune d'oeuf a été testé. 10 ml de jaune d'oeuf homogénéisé dilué au 5-10ème, placés dans un flacon, ont été soumis à un choc thermique (c.t.) en plaçant le flacon pendant 30 secondes dans un bain d'eau à 80°C.

Le diamètre des particules (D(v, 0,9) en micromètres) a été mesuré avant et après le choc thermique. Les résultats sont mentionnés sur le tableau 3 suivant.

**TABLEAU 3**

| D | N° Lec. | 0,5% de Lec. | 0,5% de Lec. modifiée | 1% de Lec. | 1% de Lec. modifiée | 2% de Lec. | 2% de Lec. modifiée |
|---|---|---|---|---|---|---|---|
| Avant c.t. | 28 | 18 | 11 | 9 | 4 | 4 | 6 |
| Après c.t. | 75 | 60 | 41 | 55 | 17 | 15 | 13 |

Il est possible d'observer que les échantillons renfermant la lécithine modifiée conforme à la présente invention étaient plus stables. En général, il est possible d'affirmer que le même effet stabilisant de la lécithine peut être obtenu avec la moitié de la quantité de lécithine modifiée conforme à la présente invention.

### Exemple 5

Une mayonnaise entière constituée de 50 g de jaune d'oeuf, 3 g de sel, 5 g de moutarde, 200 g d'huile végétale, 3 g de vinaigre, 5 ml de jus de citron et 6 ml d'eau a été produite sans lécithine ou avec différentes quantités de lécithine ordinaire ou modifiée ajoutées. 100 g de chaque mayonnaise ont été introduits dans des récipients, qui ont été obturés hermétiquement, et une incubation a été conduite pendant deux périodes de 30 minutes à 100°C. L'huile à la partie supérieure a été ensuite décantée totalement et mesurée. Les résultats sont représentés sur le tableau 4 suivant.

**TABLEAU 4**

| Echantillon | Quantité d'huile à la partie supérieure après traitement thermique, g |
|---|---|
| Aucune quantité de lécithine ajoutée | 13,2 |
| Addition de 1 % de lécithine ordinaire | 6 |
| Addition de 1,5 % de lécithine ordinaire | 5 |
| Addition de 1 % de lécithine modifiée comme dans l'exemple 1 | 3,5 |
| Addition de 2 % de lécithine modifiée comme dans l'exemple 1 | 1,1 |
| Addition de 1 % de lécithine modifiée comme dans l'exemple 2 | 1 |
| Addition de 2 % de lécithine modifiée comme dans l'exemple 2 | 0,5 |
| Addition de 1 % de lysolécithine | 2,2 |
| Addition de 2 % de lysolécithine (Emulfluid (TM), Lukas Meyer) | 1 |

Il a été de nouveau observé qu'une quantité égale ou inférieure à approximativement la moitié de la lécithine modifiée conformément à la présente invention avait le même effet stabilisant qu'une quantité comparable de lécithine ordinaire et avait même un meilleur effet stabilisant qu'une lysolécithine du commerce.

### Exemple 6

La lécithine ordinaire ou modifiée (comme dans l'exemple 2) a été dissoute dans de l'hexane, respectivement, et a été ensuite pulvérisée sur de la poudre de lait, tout en mélangeant. L'hexane a été ensuite évaporé sous agitation.

Pour la mesure de la mouillabilité, 5 g de poudre de lait ont été versés à la cuillère sur 100 ml d'eau à température ambiante et le temps d'immersion totale de la poudre de lait sous la surface a été mesuré. Les temps obtenus indiquent que la lécithine ordinaire améliore considérablement la mouillabilité de la poudre de lait, mais il a été également constaté que la lécithine modifiée conformément à l'exemple 2 présente un comportement encore meilleur.

Les résultats des temps de submersion sont mentionnés sur le tableau 5 suivant.

**TABLEAU 5**

| Echantillon | Temps de submersion, s |
|---|---|
| Poudre de lait pure | >120 |
| Poudre de lait + 0,5 % de lécithine | 21 |
| Poudre de lait + 0,5 % de lécithine modifiée | 9 |
| Poudre de lait + 1 % de lécithine | 15 |
| Poudre de lait + 1 % de lécithine modifiée | 7 |

### Exemple 7

D'une manière analogue à celle de l'exemple 6, une préparation de cacao instantané contenant du saccharose, de la poudre de cacao, de l'arôme de cacao et 1 % de lécithine a été testée. Deux échantillons contenant 1 % de lécithine ordinaire ou de la lécithine modifiée avec de la triacétine (comme dans l'exemple 2), respectivement, ont été préparés.

Les échantillons ont été soumis à une agglomération à la vapeur d'eau et mis sous forme d'une préparation de cacao du commerce. Pour l'évaluation de la mouillabilité, 21 g de la préparation de cacao ont été ajoutés sur 250 ml de lait à température ambiante et le temps de submersion de la totalité de la substance a été mesuré. Les échantillons ont été observés pendant plusieurs jours. Les résultats sont présentés sur le tableau 6 suivant.

**TABLEAU 6**

| Temps de submersion en secondes (jours) | Lécithine ordinaire | Lécithine modifiée |
|---|---|---|
| 0 | 15 | 11 |
| 3 | 35 | 17 |
| 5 | 32 | 13 |
| 7 | 35 | 10 |
| 10 | 40 | 15 |
| 21 | 35 | 12 |
| 28 | 37 | 16 |
| 35 | 42 | 19 |
| 42 | 39 | 17 |

### Exemple 8

Du beurre clarifié ODELL'S (TM), ayant une teneur en humidité d'au plus 0,1 %, a été mélangé à de la lécithine en un rapport égal à 2:1 et des lipases et phospholipases immobilisées ont été ajoutées comme dans les exemples précédents. Les conditions réactionnelles étaient également celles décrites précédemment. La lécithine modifiée obtenue avait la composition en acides gras indiquée sur le tableau 7 suivant.

**TABLEAU 7**

| Acide gras | Lécithine modifiée |
|---|---|
| C 4:0 | 1,2 |
| C 6:0 | 0,8 |
| C 8:0 | 1,5 |
| C 10:0 | 1,4 |
| C 12:0 | 3 |
| C 14:0 | 11,9 |
| C 16:0 | 20,9 |
| C 18:0 | 22,4 |
| C 18:1 | 17,9 |
| C 18:2 | 15,3 |
| C 18:3 | 3 |

## Revendications

1. Procédé d'échange de groupes acyle dans un phospholipide par échange enzymatique avec un triaclyglycérol, caractérisé en ce que la réaction s'effectue en l'absence de solvant avec un système enzymatique consistant en un mélange d'une lipase immobilisée et d'une phospholipase immobilisée, et que le triacylglycérol contient des groupes acyle saturés à chaîne courte en C2 à C4 et/ou moyenne en C6 à C12.

2. Procédé suivant le revendication 1, caractérisé par le fait que le triacylglycérol est la triacétine, l'huile de beurre ou un triglycéride en C6 à C12.

3. Procédé suivant la revendication 2, caractérisé part le fait que le triacylglycérol est la triacétine et que le phospholipide est séparable de la triacétine n'ayant pas réagi, notamment par décantation.

4. Procédé suivant la revendication 1, caractérisé par le fait que la réaction a lieu en présence d'une lécithine déshumidifiée.

## Patentansprüche

1. Verfahren zum Austausch von Acylgruppen in einem Phospholipid durch enzymatischen Austausch mit einem Triacylgylcerin, dadurch gekennzeichnet, daß man die Reaktion in Abwesenheit eines Lösemittels mit einem Enzymsystem bewirkt, das aus einer Mischung aus einer immobilisierten Lipase und einer immobilisierten Phospholipase besteht, und daß das Triacylglycerin gesättigte Acylgruppen mit kurzer Kette von C2 bis C4 und/oder mittlerer Kette von C4 bis C12 enthält.

2. Verfahren nach Anspruch 1, dadurch gekennzeichnet, daß das Triacylglycerin Triacetin, Butteröl oder ein Triglycerid mit C6 bis C12 ist.

3. Verfahren nach Anspruch 2, dadurch gekennzeichnet, daß das Triacylglycerin Triacetin ist und daß das Phospholipid von dem nicht abreagierten Triacetin abtrennbar ist, insbesondere durch Dekantieren.

4. Verfahren nach Anspruch 1, dadurch gekennzeichnet, daß die Reaktion in Gegenwart eines entfeuchteten Lecithins stattfindet.

## Claims

1. Process for exchanging acyl groups in a phospholipid by enzyme exchange with a triacylglycerol, characterised in that the reaction is performed in the absence of solvent with an enzyme system consisting of a mixture of an immobilised lipase and an immobilised phospholipase, and that the triacylglycerol contains saturated short-chain C2 to C4 and/or medium-chain C6 to C12 acyl groups.

2. Process according to claim 1, characterised in that the triacylglycerol is triacetin, butter oil or a C6 to C12 triglyceride.

3. Process according to claim 2, characterised in that the triacylglycerol is triacetin and that the phospholipid may be separated from the unreacted triacetin, in particular by decantation.

4. Process according to claim 1, characterised in that the reaction takes place in the presence of a dried lecithin.
